# EUROPEAN PATENT APPLICATION

(11) **EP 4 465 304 A1**
(43) Date of publication of application: **20.11.2024**
(21) Application number: 22920122.3
(22) Date of filing: 12.12.2022
(51) Int. Cl.: G16H 50/00, A61B 5/15, G16H 50/50

(54) **METHOD AND SYSTEM FOR PREDICTING GLUCOSE VALUES AND GENERATING HYPOGLYCAEMIA AND HYPERGLYCAEMIA WARNINGS**

(30) Priority: 12.01.2022 ES 202230016
(71) Applicant: Universidad Complutense De Madrid, 28040 Madrid (ES); Universidad De Extremadura, 06006 BADAJOZ (ES)
(72) Inventor: HIDALGO PÉREZ, José Ignacio, 28223 Madrid (ES); HIDALGO GARCÍA, Javier, 28018 Madrid (ES); LANCHARES DÁVILA, Juan, 40410 SEGOVIA (ES); ALVARADO DÍAZ, Jorge, 06480 Badajoz (ES); VELASCO CABO, José Manuel, 28939 Madrid (ES); GARNICA ALCÁZAR, Óscar, 28420 Madrid (ES); FERNÁNDEZ DE VEGA, Francisco, 06800 BADAJOZ (ES); CHÁVEZ DE LA O, Francisco, 06800 BADAJOZ (ES)
(74) Representative: Balder IP Law, S.L.
(86) International application number: PCT/ES2022/070789
(87) International publication number: WO 2023/135346

(57) **Abstract**

Blood glucose monitoring is a difficult task that people with diabetes often have to perform on their own. Accurate and timely prediction is vital for making decisions and recommending corrective actions. Therefore, there is a need to develop effective and accurate glucose prediction methods that allow the development of safe blood glucose monitoring systems using simple and convenient devices for the patient.

The present invention describes a non-invasive method and system for the prediction of glucose values, based on the estimation from variables measured with an activity wristband. The system uses variables that are not directly related to glucose to estimate and predict glucose values and generates alerts to dangerous situations of hypoglycemia and hyperglycemia.

## Description

### TECHNOLOGY SECTOR

This invention is framed in the field of automatic control of biological systems. More specifically, it describes a device and a method for the personalized prediction of the blood glucose level to prevent hypoglycemia and hyperglycemia being, therefore, of application in Medicine.

### BACKGROUND OF THE INVENTION

Diabetes is a chronic disease that is irreversible and affects the transformation of sugar into energy. It occurs when the concentration of glucose in the blood (glycemia) is too high due to insufficient insulin production or incorrect use of insulin by the body, so that glucose remains in the bloodstream and does not reach the cells.

Blood glucose monitoring is a difficult task that people with diabetes often have to perform on their own. In addition, accurate and timely prediction is vital for making decisions and recommending corrective actions to the patient when the future blood glucose value is out of the target range. Events such as hyperglycemia and hypoglycemia can cause severe short-term health damage and potential long-term permanent damage, so it is crucial to prevent such events in order to avoid them.

To measure glucose levels, the diabetic patient usually has a hand-held meter (glucometer) or a continuous monitoring system to make a decision about injecting insulin or eating food. This is a tedious task that is performed daily by the patient or caregiver and causes extra stress. Therefore, other methods of measuring and monitoring glucose levels have been investigated and tested, with those based on glucose level prediction being of particular interest, which may allow patients to anticipate when there is a risk of hypoglycemia or hyperglycemia.

The glucose-insulin system has a non-linear behavior. The amount of glucose in the blood is a dynamic value that depends on a large number of factors such as diet, physical exercise, sleep pattern, etc. Up to 42 factors have been described that influence blood glucose, which, in addition, have an evolutionary variability in glucose. All this makes the prediction of blood glucose levels a very difficult task. Moreover, predictions must be very accurate, since a wrong dose of insulin, calculated by an erroneous prediction, can cause a health problem for the patient. In this sense, the contribution of *Data Mining* and *Machine Learning* has been fundamental in recent years, since they allow classifying, analyzing and validating data to extract knowledge and identify patterns autonomously and continuously. These tools are used in glucose prediction, both for the prediction of the glucose profile and in the prediction of the glycemic response to the variables analyzed. This has given rise to glucose predictor applications (DIABITIS, JANUARY Al, QUIN, PredictBGL) that allow reporting the effect on glucose of a given action, such as the ingestion of a particular food or predicting nocturnal hypoglycemia. Each of them has a functionality but all of them have limitations and, in general, little safety data (Patricia Enes Romero, Nuevas Apps predictoras de glucosa, Revista DIABETES N° 69, May-June 2021).

In this sense, different automation approaches to control diabetes have been tested, such as the development of an "artificial pancreas", which is a closed-loop system comprising a continuous glucose sensor, a control algorithm and an insulin pump, with an advanced prediction system that allows insulin to be injected automatically as a healthy person's pancreas would. Some techniques studied for this prediction model are: *Genetic Programming* (WO2015079079, ES2845400T3), *Random Forest, k-Nearest Neighbor* algorithms, *Grammatical Evolution, Fuzzy Rules Based Systems* (WO2016110745, US7395158, US20120120123234), the most promising being those based on neural networks (*Neuronal Networks,* NN).

However, there is still a need to develop methods for the efficient and accurate prediction of blood glucose values that will allow the development of safe blood glucose monitoring systems using simple and patient-friendly devices.

### EXPLANATION OF THE INVENTION

The present invention describes a method and a system for the prediction of glucose values and generation of hypoglycemia and hyperglycemia alerts, based on the estimation of the interstitial glucose value from variables measured with an activity wristband. A method of modeling the blood glucose level using image generation, *wavelet* transforms and deep learning is also described. The system uses variables that are not directly related to glucose to perform glucose level estimation by non-invasive methods. A third aspect of the invention refers to the generation of alerts in case of dangerous situations due to alterations in glucose levels.

The aim of the system is to predict non-invasively and safely whether the patient will suffer a hypoglycemia or hyperglycemia event in the next 24 hours.

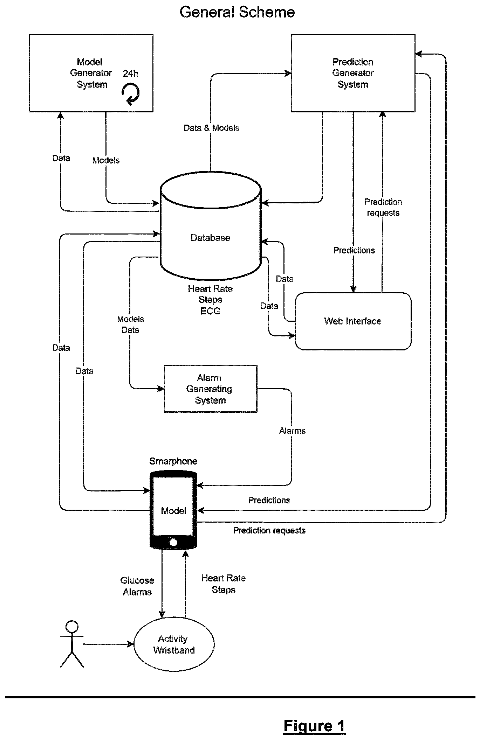

The system (Figure 1) comprises:
- An activity wristband that collects heart rate, physical activity, energy expenditure and electrocardiogram (ECG) data,
- A database
- A prediction generator module
- A glucose model generator module
- A physiological variable generator module
- An alarm generator module
- A pattern analyzer
- A web interface
- A mobile device or Tablet with internet connection, which stores the information collected by the activity wristband, interfaces with the database and other blocks of the system; and stores local models and generates alarms in the activity wristband.

The system collects patient data through a smart bracelet device that collects variables such as heart rate or activity performed, among others, and these data are stored in the database and used by the device to generate predictive models and generate warnings, as well as recommendations to provide solutions to possible inconveniences that the patient may encounter.

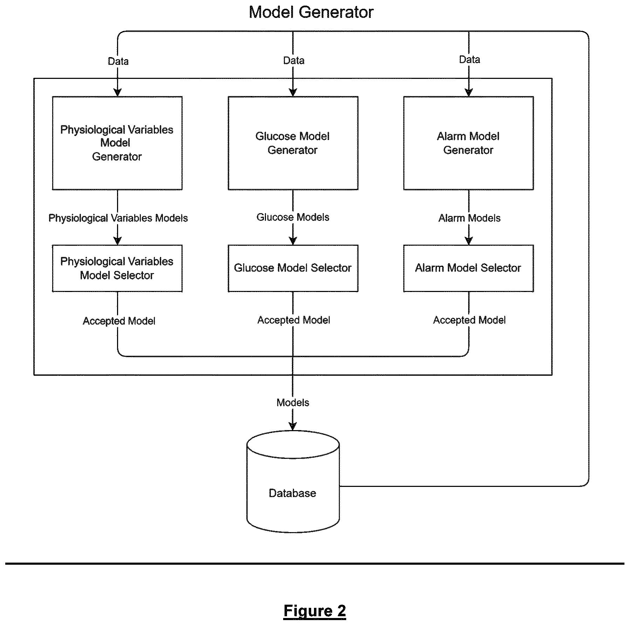

The main objective of the prediction model generator (Figure 2) is to develop "generic" models that are adaptable and usable by users whose data have not been used in the training process. These models are trained in a previous phase using available data previously collected from volunteer users, including interstitial glycemia data. Although these models may use for prediction data collected by a continuous glucose meter, in the present invention the data is collected through an activity wristband worn by the user, in a non-invasive manner. The combined use of *wavelet* Transforms, *Deep Learning,* Genetic Programming, Evolutionary Grammars and Fuzzy Logic Based Systems, confer a predictive potential not available in the systems currently in use, allowing the generation of warning alarms to the user to enable behavioral changes necessary to avoid hypo- and hyperglycemia events.

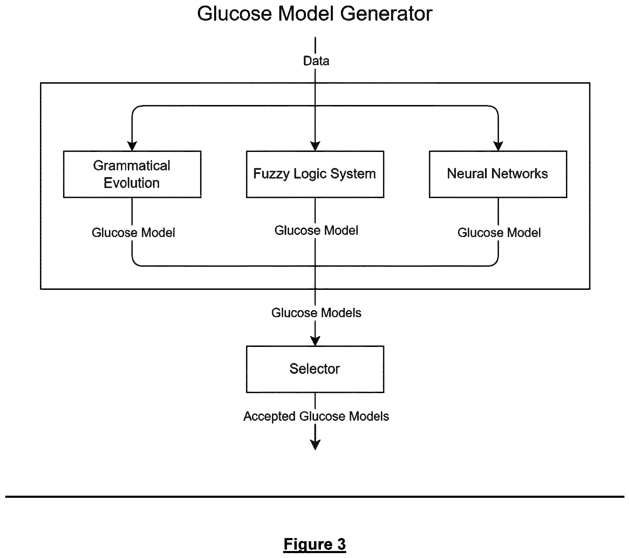

The glucose model generator (Figure 3) is a system that, using glucose data measured with a continuous glucose monitor and data from an activity wristband or other devices, generates predictive models to estimate the blood glucose value from these data. Only those models that meet the accuracy thresholds are accepted for inclusion in the set of models available and visible to the user. There are three subsystems for glucose model generation depending on the artificial intelligence technique used:
- Grammatical Evolutio Module. Generates models using genetic programming techniques
- Neural networks module. Generates models using deep learning techniques.
- Module of systems based on fuzzy logic. It performs the prediction of blood glucose values using a system based on Takagi-Sugeno-Kang fuzzy rules in two phases. The first phase is a learning process in which membership functions and rules are optimized using a genetic algorithm. In the second phase of tuning, a genetic algorithm is used to perform rule selection and optimization.

The glucose models are trained using two different scenarios: *What-if* and *Agnostic.* In both, the inputs are values measured by a continuous glucose monitoring system as well as previous carbohydrate intake and insulin injections. In the *What-if* scenario*,* assumed future values of meals and insulin injections are supported; on the other hand, in the *Agnostic* system, only information from past and present events is available for prediction.

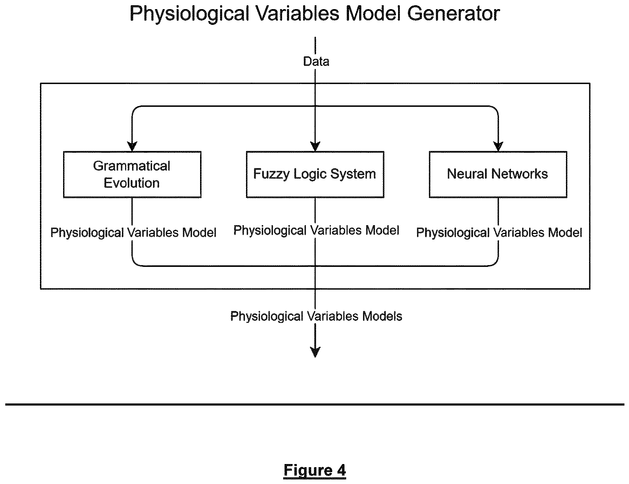

The physiological variable model generator (Figure 4) works in a similar way to the glucose models, but using the physiological variables.

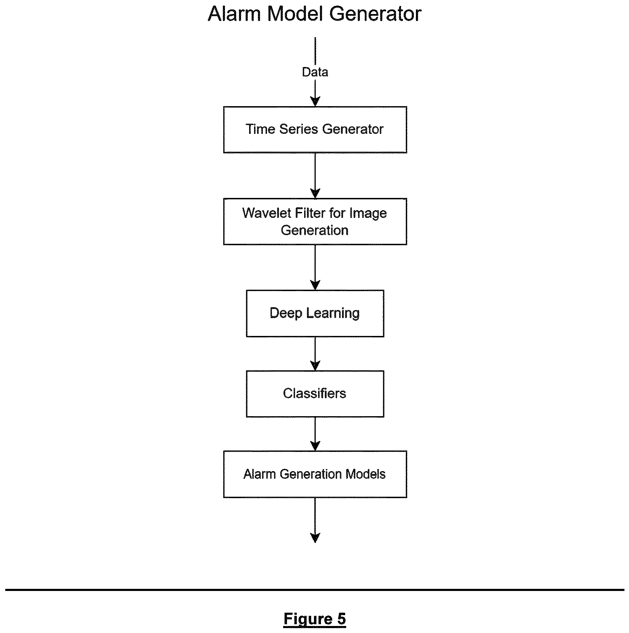

The alarm model generator (Figure 5) takes data obtained from the continuous glucose meter and generates a time series to which a *wavelet* filter is applied to generate images. These images are used to train a deep learning classification system. Images obtained with time series that correspond to situations of hypoglycemia or hyperglycemia in the following 24 hours are detected and generate an alarm signal that is sent to the mobile device or Tablet and it triggers the alarm.

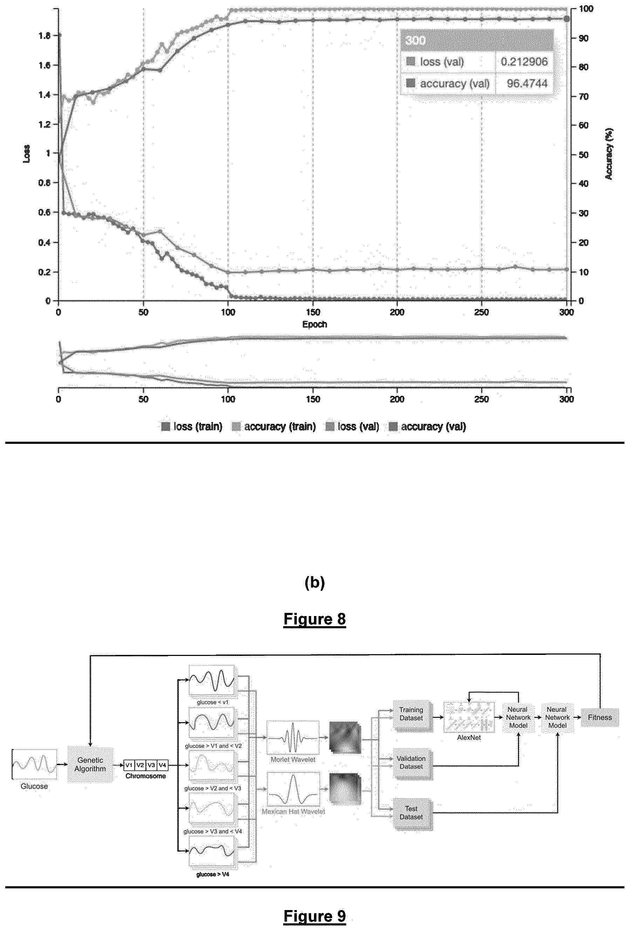

To the generation of images from the time series data, a data enhancement phase with a *rolling window* of 1 hour is added and then a wavelet transform is applied with the *Mexican Hat* function and the *Morlet* function. Thus, spectrograms are generated, differentiating between those from data in which there are events classified into five categories: severe hypoglycemia, hypoglycemia, normoglycemia, hyperglycemia and severe hyperglycemia). These levels are adjusted in a personalized way using Fuzzy logic and evolutionary algorithms (Figure 9). This generates four classification models that generate signals over the next 24 hours.

### BRIEF DESCRIPTION OF THE DRAWINGS

To complement the description being made and for a better understanding of the features of the invention, there is attached as an integral part of said description, a set of drawings wherein, illustratively and non-limitingly, the following has been depicted:
Figure 1. General scheme of the system and method for predicting glucose values and generating hypoglycemia and hyperglycemia alerts.
Figure 2. Schematic of the system prediction model generator.
Figure 3. Schematic of the glucose model generator of the system.
Figure 4. Diagram of the model generator of physiological variables.
Figure 5. Schematic of the alarm model generator.
Figure 6. Glucose monitoring record and spectrogram
Figure 7. Neural network training with data from all patients using *Mexican Hat wavelet* (a) and using *Morlet wavelet* (b).
Figure 8. Training of the neural network (2) using the data of one patient for testing and training the network with the rest of the patients, using *Mexican Hat wavelet* (a) and using *Morlet wavelet* (b).
Figure 9. Schematic of the adjustment of hypoglycemia, normoglycemia, hyperglycemia and severe hyperglycemia levels using Fuzzy logic and evolutionary algorithms.

### PREFERRED EMBODIMENT OF THE INVENTION

The present invention is illustrated by the following example, which is not intended to be limiting in scope.

### Example 1

This example refers to a system for predicting hypoglycemia events using wavelets and convolutional neural networks.

An activity wristband collects heart rate, physical activity, energy expenditure and electrocardiogram (ECG) data from a subject and these data are stored in a database.

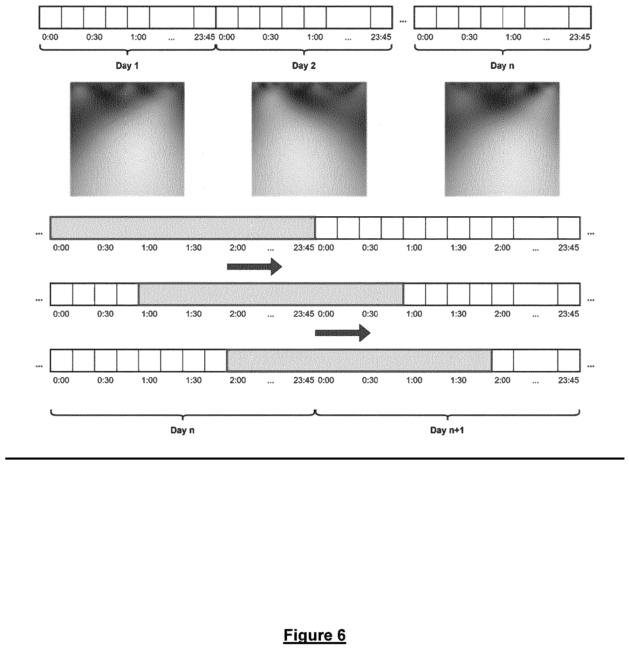

A continuous glucose monitoring (CGM) system obtains recordings every few minutes (e.g., 5-15 minutes) for 23 real patients with type 1 diabetes (Figure 6).

A cell phone with internet connection stores the information collected by the activity wristband, interfaces with the database, the rest of the system modules, stores local models and generates alarms in the activity wristband.

To train the convolutional neural network, spectrograms (96 records per day/spectrogram) are generated for each patient using *Continuous Wavelet Transform* (CWT) (Figure 6). Based on 96 glucose records per day for each patient, the spectrograms are classified as:
- Normal: if glucose values are maintained above 55 mg/dL.
- Hypoglycemia: if glucose values are below 55 mg/dL.

Using the *data augmentation* technique, the number of images per patient is increased. Spectrograms are generated by running through all the glucose values using a *rolling window* of size 1 day (96 records) with a 1-hour offset. Using the NVIDIA DIGITS *framework* with *TensorFlow,* the images are classified (Figure 9).

The *datasets* are organized as follows:
Training: 85% Training: 85% Training: 85
Validation:10% Validation:10% Validation:10% Validation:10% Validation:10% Validation:10% Validation
Test: 5% Test: 5% Test: 5% Test: 5% Test: 5% Test: 5% Test: 5% Test: 5%

*The AlexNet* neural network is used to train the models using the following training parameters:
*Learning rate*: 0.001
Number of epochs: 300
Validation interval (epochs): 10
Optimization algorithm: stochastic gradient descent
Image *crop* size: 227 pixels

Two types of experiments are performed: (1) training the network with data from all patients; (2) using data from one patient for testing and training the network with the rest of the patients. For each experiment, two *wavelets* are used within the CWT: *Mexican Hat* and Morlet.

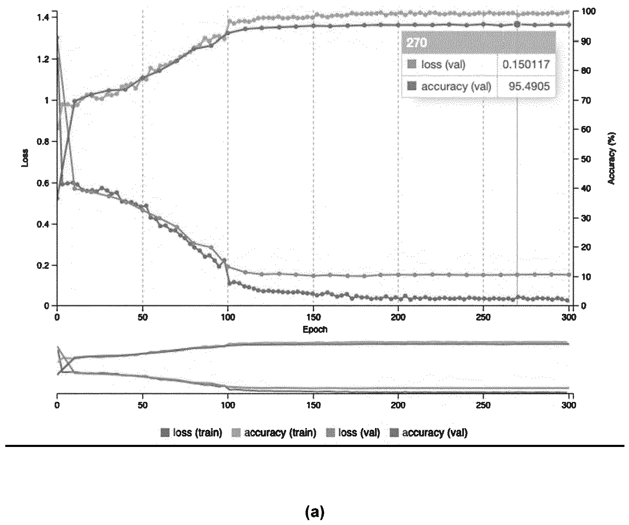

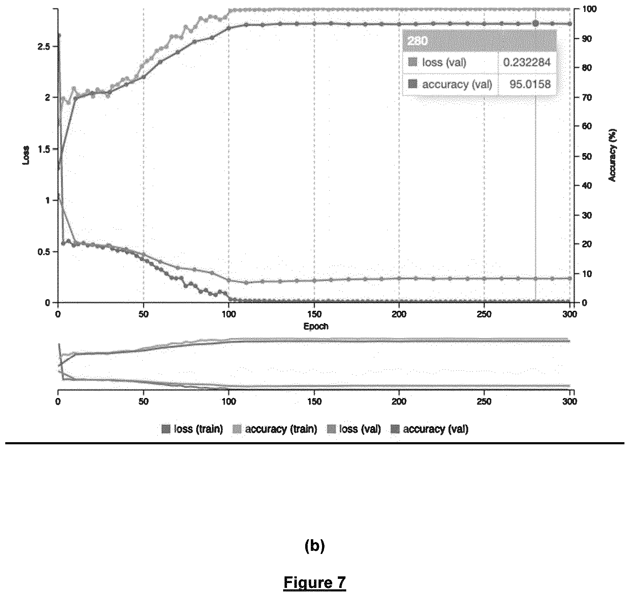

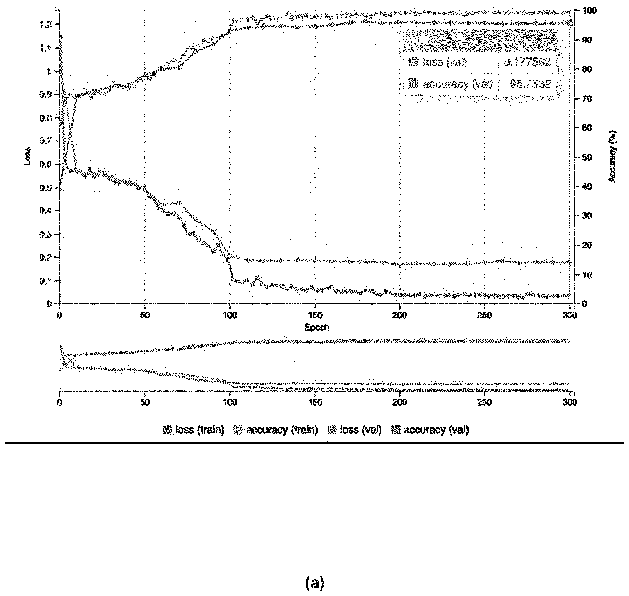

Figure 7 shows the results for experiment (1) using *Mexican Hat wavelet* (a) and using *Morlet wavelet* (b). Figure 8 shows the results for experiment (2) using *Mexican Hat wavelet* (a) and using *Morlet wavelet* (b).

As a result, the trained models are quite accurate, with hit rates above 95% in the validation phase but the models do not correctly classify images with untrained patient data, so training is required.

## Claims

1. System for prediction of glucose values and generation of hypoglycemia and hyperglycemia alerts comprising:
- An activity wristband that collects heart rate, physical activity, energy expenditure and electrocardiogram (ECG) data,
- A database
- A prediction generator module
- A glucose model generator module
- A physiological variable generator module
- An alarm generator module
- A pattern analyzer
- A web interface
- A mobile device or Tablet with internet connection, which stores the information collected by the activity wristband, interfaces with the database and other blocks of the system; and stores local models and generates alarms in the activity wristband,
**characterized in that** the alarm model generator takes data obtained from a continuous glucose meter and obtains a time series to generate images corresponding to hypoglycemia or hyperglycemia situations, which are used to train a learning system, to which a data augmentation phase is added with a *rolling window* and then a wavelet transform is applied with the *Mexican Hat* function and with the Morlet function.

2. System according to claim 1, wherein the prediction models are trained using available data previously collected from volunteers, including interstitial blood glucose data.

3. System according to claim 1, wherein the glucose models are generated using different artificial intelligence techniques such as genetic programming, deep learning and Takagi-Sugeno-Kang fuzzy rules.

4. System according to claim 3, wherein the glucose models are trained using *What-if* and *Agnostic* scenarios.

5. System according to claim 1, wherein the spectrograms generated in the alarm models correspond to five categories: severe hypoglycemia, hypoglycemia, normoglycemia, hyperglycemia and severe hyperglycemia.

6. System according to claim 5, wherein alarm signals are generated for the four categories other than normoglycemia.

7. Non-invasive method for predicting glucose values and generating hypoglycemia and hyperglycemia alerts using the claimed system comprising:
- Store a user's physiological variables and interstitial glucose data in the activity wristband.
- Generate prediction models by measuring interstitial glucose and physiological variables in volunteer individuals different from the user.
- Generate blood glucose models from user interstitial glucose data.
- Generate models of user physiological variables.
- Generate hypoglycemia and hyperglycemia alarm models.
**characterized by** the alarm model generator which takes data obtained from a continuous glucose meter and obtains a time series to generate images corresponding to hypoglycemia or hyperglycemia situations, which are used to train a learning system, to which a data enhancement phase is added with a *rolling window* and then a wavelet transform is applied with the *Mexican Hat* function and with the Morlet function, generating spectrograms.

8. Non-invasive method according to claim 7, wherein the physiological variable data is taken using an activity wristband worn by the user.

9. Non-invasive method according to claim 7, wherein the prediction models are trained using available data previously collected from volunteers, including interstitial blood glucose data.

10. Non-invasive method according to claim 7, wherein the glucose models are generated using different artificial intelligence techniques such as genetic programming, deep learning and Takagi-Sugeno-Kang fuzzy rules.

11. Non-invasive method according to claim 10, wherein the glucose models are trained using *What-if* and *Agnostic* scenarios.

12. Non-invasive method according to claim 7, wherein the spectrograms generated in the alarm models correspond to five categories: severe hypoglycemia, hypoglycemia, normoglycemia, hyperglycemia and severe hyperglycemia.

13. Non-invasive method according to claim 12, wherein alarm signals are generated for the four categories other than normoglycemia.
